# EUROPÄISCHE PATENTANMELDUNG

(11) **EP 4 242 624 A1**
(43) Veröffentlichungstag der Anmeldung: **13.09.2023**
(21) Anmeldenummer: 22161384.7
(22) Anmeldetag: 10.03.2022
(51) Int. Cl.: G01N 1/31, G01N 1/42, G01N 35/04, G01N 23/00, G01N 35/10, H01J 37/00, G01N 23/20025

(54) **VERFAHREN UND VORRICHTUNG ZUM BEREITSTELLEN VON BIOLOGISCHEN PROBEN IN EINEM VITRIFIZIERTEN ZUSTAND FÜR STATISCHE UND ZEITAUFGELÖSTE STRUKTURUNTERSUCHUNGEN MIT HILFE VON ELEKTRONEN- ODER RÖNTGENQUELLEN**

(71) Anmelder: Max-Planck-Gesellschaft zur Förderung der Wissenschaften e.V., 80539 München (DE)
(72) Erfinder: TELLKAMP, Friedjof, 22761 Hamburg (DE); SCHULZ, Eike-Christian, 22761 Hamburg (DE); SCHIKORA, Hendrik, 22761 Hamburg (DE); MEHRABI, Pedram, 22761 Hamburg (DE); KOLLEWE, Martin, 22761 Hamburg (DE)
(74) Vertreter: v. Bezold & Partner Patentanwälte - PartG mbB

(57) **Zusammenfassung**

Die Erfindung betrifft ein Verfahren zum Vitrifizieren einer biologischen Probe (1). Das Verfahren umfasst ein Positionieren eines Probenträgers (2) mit der biologischen Probe (1) mittels einer Transfereinrichtung (12) in einer Ausgangsposition (P₁). Hierbei weist der Probenträger (2) einen Sockel (2a) und einen, vom Sockel (2a) entlang einer Trägerachse (A) abstehenden, Stift (2b) auf. Weiterhin ist die biologische Probe (1) am Stift (2b) beabstandet vom Sockel (2a) befestigt. Das Verfahren umfassen ferner ein Zuführen einer Flüssigkeit zu der biologischen Probe (1) in der Ausgangsposition (P₁) mittels eines Flüssigkeitsdispensers (13). Weiterhin umfassen das Verfahren ein Bewegen des Probenträgers (2) mit der biologischen Probe (1) mittels der Transfereinrichtung (12) entlang eines vorbestimmten Transferpfads (T) von der Ausgangsposition (P₁) in eine Freigabeposition (P₂), wobei die biologische Probe (1) in der Freigabeposition (P₂) in oder angrenzend zu einem verflüssigten Gas (3) angeordnet ist. Hierbei ist vorgesehen, dass der Transferpfad (T) zur Trägerachse (A) geneigt oder entlang eines Kreisbogens verläuft. Weiterhin betrifft die Erfindung eine Vitrifizierungsvorrichtung 10, die zur Durchführung des Verfahrens ausgebildet ist.

## Beschreibung

### Technisches Gebiet

Die Erfindung betrifft Verfahren zum Vitrifizieren biologischer Proben. Weiterhin betrifft die Erfindung eine Vitrifizierungsvorrichtung, die zur Durchführung eines der Verfahren ausgebildet ist. Anwendungsmöglichkeiten der Erfindung liegen in der Bereitstellung von biologischen Proben (z. B. von Proteinkristallen) in einem vitrifizierten Zustand für statische und/oder zeitaufgelöste Strukturuntersuchungen mithilfe von Elektronen- oder Röntgenquellen.

### Stand der Technik

In der vorliegenden Anmeldung wird auf folgende Dokumente Bezug genommen, welche den technischen Hintergrund der Erfindung verdeutlichen:
[1] SPINE SAMPLE HOLDER & VIAL SPECIFICATIONS-L-R05 (15. April 2014);
[2] P. Mehrabi et al., "Liquid application method for time-resolved analyses by serial synchrotron crystallography", Nat. Methods 16, 979-982 (2019);
[3] P. Mehrabi et al., "Time-resolved crystallography reveals allosteric communication aligned with molecular breathing", Science 365, 1167-1170 (2019);
[4] E.C. Schulz et al., "The hit-and-return system enables efficient time-resolved serial synchrotron crystallography", Nat. Methods 15, 901-904 (2018);
[5] P. Mehrabi et al., "The HARE chip for efficient time-resolved serial synchrotron crystallography", J. Synchrotron Rad. 27, 360-370 (2020);

Zeitaufgelöste Untersuchungen an Synchrotronstrahlungsquellen oder Elektronmikroskopen sind wichtige Methoden, um die Struktur von biologischen Proben und insbesondere deren Dynamik bestimmen zu können. Die hierfür hauptsächlich verwendete Methode ist die Beugung von Synchrotronstrahlung oder kohärenten Elektronen, aus deren Beugungsbildern sich die Struktur rekonstruieren lässt. Zu diesem Zwecke müssen die biologischen Proben, in erster Linie Proteine, in kristalliner Form vorliegen und in den Strahl, z. B. der Synchrotronstrahlung, gebracht werden.

Eine etablierte Methode zur Untersuchung von Proteinkristallen an Synchrotronstrahlungsquellen ist die Aufbringung einzelner oder mehrerer mikroskopischer Kristalle (z. B. mit typischen Querschnittsdimensionen im Bereich 10 µm bis 100 µm) auf eine spezielle Art von Probenträger. Diese durch den SPINE-Standard [1] definierten und von verschiedenen Herstellern vertriebenen Probenträger weisen einen Sockel (engl. "cap") auf, der als Halterung für einen, vom Sockel abstehenden, Stift (engl. "pin") dient. Ein oder mehrere zu untersuchende Proteinkristalle werden am freien Ende des Stifts (z. B. mittels eines Netzchen oder einer mikroskopisch kleinen Schleife) befestigt und so im Strahl vermessen.

Da Proteinkristalle zu großem Teil aus Wasser bestehen, müssen diese zwischen ihrer Präparation und der Beugungsuntersuchung vor einem Austrocknen geschützt werden. Dies geschieht üblicherweise per Einfrieren bzw. Vitrifikation, d. h. durch ein sehr schnelles Abkühlen (Schockgefrieren) der Kristalle (z. B. in flüssigem Stickstoff oder Ethan). Die so gefrorenen Proben können auf diese Art zeitlich und räumlich unabhängig von der Verfügbarkeit einer Messung an einer Synchrotronstrahlungsquelle herstellt werden. Es ist üblich, mehrere mit Kristallen bestückte Probenträger in einer dafür vorgesehenen Aufbewahrungsvorrichtung zu lagern und/oder an eine Synchrotronstrahlungsquelle zu verschicken. Viele Synchrotronstrahlungsquellen weltweit benutzen standardisierte Geometrien für diese Aufbewahrungsvorrichtungen bzw. Probenträger, um die entsprechenden Proben dann vollautomatisiert vermessen zu können. In der Regel verbleiben die Proben dabei ab dem Zeitpunkt ihrer Vitrifikation bis hin zur Messung, d. h. auch während des Versands, in einer tiefkalten Atmosphäre.

Das vorgenannte Vorgehen erlaubt in erster Linie die Untersuchung von statischen Systemen. D. h., die Struktur der Proben ändert sich nicht über die Zeit. Um Erkenntnisse zu erlangen, wie Reaktionen und Prozesse in Proteinen ablaufen, wurden verschiedene Methoden zur zeitaufgelösten Strukturanalyse entwickelt. Bei den bislang bekannten Ansätzen zur zeitaufgelösten Untersuchung wird die Probe üblicherweise nicht eingefroren bzw. vitrifiziert, wobei auch in diesem Fall darauf geachtet werden muss, dass die Proben nicht austrocknen. Lediglich beispielhaft sei in diesem Zusammenhang die "serielle Fixed-Target Methode" [2, 3, 4] erwähnt, bei der mehrere 10000 Proben auf einen speziellen Halter aufgebracht werden und nach Anregung einer Reaktion per Licht oder dem Hinzufügen einer Flüssigkeit jeweils einzeln und mit einer bestimmten Zeitverzögerung vermessen werden. Durch Variation der Zeitverzögerung zwischen der Anregung und der Vitrifizierung über mehrere Messungen kann so die Dynamik der Struktur bestimmt werden.

Nachteilig an den bisherigen Ansätzen ist jedoch die Komplexität der mit diesen Methoden verbundenen Aufbauten. Nachteilig ist weiterhin, dass die Präparation der Proben zeitlich und räumlich nicht von der Messung an einer Synchrotronstrahlungsquelle getrennt werden kann. D. h., bei den bisherigen Ansätzen müssen die Proben kurz vor einer Messung an einer Synchrotronstrahlungsquelle präpariert werden [5].

Für zeitaufgelöste Untersuchungen an Elektronmikroskopen, bei welchen typischerweise eine andere Art von Probenträger verwendet wird, existieren Ansätze, eine Reaktion der Probe per Lichtimpuls oder dem Hinzufügen einer Flüssigkeit zu starten und dann nach einer gewissen Zeitverzögerung durch Einfrieren bzw. Vitrifizieren zu stoppen. Durch die Herstellung mehrerer Proben, die mit unterschiedlichen Zeitverzögerungen vitrifiziert wurden, lassen sich so Erkenntnisse über die Dynamik der Reaktion erhalten. Aus der Praxis sind dazu Ansätze bekannt, mit welchen man diesen Prozess halbautomatisiert bzw. reproduzierbar ablaufen lassen kann, jedoch nur für Probenträger, wie sie für Messungen in Elektronmikroskopen benutzt werden.

Ein Vitrifizieren von Proben für Messungen an Synchrotronstrahlungsquellen mittels der vorgenannten Geräte ist dabei nicht problemlos möglich, da die entsprechenden Geräte nicht für die Geometrie der typischerweise bei Synchrotronstrahlungsquellen verwendeten Probenträger nach dem SPINE-Standard ausgelegt sind. Dies liegt darin begründet, dass die Spitze der SPINE-Probenträger sehr viel keiner ist, als die Probenträger für Elektronmikroskope sind und daher die üblicherweise verwendeten Flüssigkeitsdispensers zum Applizieren einer Flüssigkeit auf den Proben sehr genau ausgerichtet werden müssen. Typische Abstände zwischen Probe und Flüssigkeitsdispenser liegen für SPINE-Probenträger hierbei im Bereich von ca. 1 mm oder weniger. Entsprechen hoch ist die Gefahr eines Kollidierens des Probenträger mit dem Flüssigkeitsdispenser.

### Aufgabe der Erfindung

Aufgabe der Erfindung ist es daher, eine verbesserte Möglichkeit zum Einfrieren bzw. Vitrifizieren von biologischen Proben für zeitaufgelöste Messungen an Synchrotronstrahlungsquellen bereitzustellen. Bevorzugt ist es dabei eine Aufgabe der Erfindung, eine entsprechende Möglichkeit bereitzustellen, mittels derer auf einen SPINE-Probenträger gehalterte biologische Proben nach dem Auslösen einer Reaktion in der biologischen Probe möglichst automatisiert und reproduzierbar vitrifiziert werden können.

### Beschreibung der Erfindung

Diese Aufgaben werden durch Verfahren und eine Vorrichtung mit den Merkmalen der unabhängigen Ansprüche gelöst. Vorteilhafte Ausführungsformen und Anwendungen der Erfindung sind Gegenstand der abhängigen Ansprüche und werden in der folgenden Beschreibung unter teilweiser Bezugnahme auf die Figuren näher erläutert.

Gemäß einem ersten unabhängigen Aspekt der Erfindung wird ein Verfahren bereitgestellt. Bevorzugt dient das Verfahren zum, vorzugsweise automatisierten, Vitrifizieren einer biologischen Probe (z. B. einem Proteinkristall). Unter dem an sich bekannten Vorgang des "Vitrifizierens" kann dabei bevorzugt ein Abkühlen der Probe unter den Gefrierpunkt verstanden werden, ohne dass sich dabei (Wasser-)Kristalle in der Probe bilden. Hierfür sind schnelle Abkühlraten (z. B. Abkühlraten größer als eine Million Grad Celsius pro Sekunde) vorgesehen, welche z. B. durch ein Eintauchen der Proben in ein Bad aus flüssigem Stickstoff oder flüssigem Ethan realisiert werden können. Entsprechend kann der Ausdruck Vitrifizieren vorzugsweise auch ein Schockgefrieren der biologischen Proben bezeichnen. Aufgrund des schnellen Abkühlens (z. B. innerhalb weniger Millisekunden) finden die Wassermoleküle keine Zeit, um Eiskristalle zu bilden, sondern sie erstarren und bilden einen festen in der Regel amorphen Körper, sodass die vitrifizierten Proben eine möglichst wirklichkeitsnahe Struktur beibehalten.

Das erfindungsgemäße Verfahren umfasst ein Positionieren eines Probenträgers mit der biologischen Probe mittels einer Transfereinrichtung in einer ersten Position, welche zur besseren Unterscheidung im Folgenden als "Ausgangsposition" bezeichnet werden kann. Hierbei ist vorgesehen, dass der Probenträger einen Sockel und einen (z. B. länglichen) Stift aufweist, wobei der Stift vom Sockel entlang einer Trägerachse absteht. Beispielsweise der Stift kann ein erstes Ende aufweisen, das am Sockel befestigt (z. B. eingesteckt) ist, und ein, dem ersten Ende entgegengesetztes, zweites Ende aufweisen, das frei ist. Der Probenträger kann somit z. B. ein nach dem SPINE-Standard ausgebildeter Probenträger sein. Weiterhin ist vorgehen, dass die biologische Probe am Stift beabstandet vom Sockel (z. B. mittels eines Netzchens oder einer mikroskopisch kleinen Schleife) befestigt ist. D. h., die biologische Probe kann in einem vorbestimmten Abstand vom Sockel am Stift (z. B. an dessen zweiten Ende) gehaltert sein.

Das Verfahren umfasst ferner ein Zuführen einer Flüssigkeit (z. B. Wasser oder eine flüssige Lösung einer Wirksubstanz) zu der biologischen Probe in der Ausgangsposition mittels eines Flüssigkeitsdispensers. D. h., bevorzugt soll in der Ausgangsposition ein Befeuchten der Probe mittels des Flüssigkeitsdispensers erfolgen. Als Flüssigkeitsdispenser kann dabei eine Vorrichtung zum kontrollierten (z. B. tröpfchenweise) Dosieren der Flüssigkeit verstanden werden. Beispielsweise kann zum Zuführen der Flüssigkeit eine Flüssigkeitsaustrittsöffnung (z. B. das Ende einer Kanüle) des Flüssigkeitsdispensers in einem Abstand von wenigen Millimetern, vorzugsweise in einem Abstand < 1 mm, von der biologischen Probe positioniert werden. Auf vorteilhafte Weise kann durch die Flüssigkeit, insbesondere die Wirksubstanz, z. B. eine chemische und/oder enzymatische, Reaktion in der biologischen Probe gestartet werden.

Das Verfahren umfasst weiterhin ein Bewegen des Probenträgers mit der biologischen Probe mittels der Transfereinrichtung entlang eines vorbestimmten Transferpfads von der ersten Position bzw. der Ausgangsposition in eine zweite Position, die im Folgenden als Freigabeposition bezeichnet werden kann. D. h., bevorzugt soll die (befeuchtete) Probe nach dem Zuführen der Flüssigkeit bzw. dem Starten einer Reaktion mittels der Transfereinrichtung von der Ausgangsposition in die Freigabeposition transferiert werden. Hierbei ist vorgesehen, dass die biologische Probe in der Freigabeposition in oder angrenzend zu einem verflüssigten Gas (z. B. flüssigem Stickstoff oder flüssigem Ethan) angeordnet ist. Entsprechend kann in der Freigabeposition ein Einfrieren bzw. Schockgefrieren der (befeuchteten) biologischen Probe erfolgen. Dies kann dabei z. B. dadurch erfolgen, dass die biologische Probe in der Freigabeposition bereits vom verflüssigten Gas umgeben ist oder dass die biologische Probe bzw. der Probenträger in der Freigabeposition aus der Transfereinrichtung ausgelöst bzw. freigegeben wird und schwerkraftvermittelt (d. h. unter der Wirkung der Schwerkraft) in das verflüssigte Gas fällt. Hierdurch kann auf vorteilhafte Weise der aktuelle Zustand der biologischen Probe (z. B. ein durch die Flüssigkeitszugabe erzeugtes Reaktionsintermediat) konserviert werden.

Das Verfahren zeichnet sich dadurch aus, dass der Transferpfad zur Trägerachse geneigt verläuft. D. h., bevorzugt erfolgt das Bewegen des Probenträgers mit der biologischen Probe schräg zur Trägerachse (z. B. mit einem Neigungswinkel zwischen 10° und 30°). Auf vorteilhafte Weise kann dadurch sichergestellt werden, dass der Probenträger beim Transferieren nicht mit dem Flüssigkeitsdispenser kollidiert. Hierdurch kann auf vorteilhafte Weise ein möglichst rasches Vitrifizieren der Proben erreicht werden, da keine Zeit für ein Zurückziehen des Flüssigkeitsdispensers zur Vermeidung einer Kollision verwendet werden muss. Ein weiterer Vorteil der kontrollierten Bewegung ist hierbei, dass so in kurzer Zeit eine hohe Anzahl von Proben (z. B. mit unterschiedlichen Zeitverzögerungen und damit Reaktionszuständen) vitrifiziert werden können und damit ein Probensatz für Synchrotronstrahlungsquellen bereitgestellt werden kann, welcher zeitaufgelöste Strukturuntersuchungen ermöglicht.

Nach einem ersten Aspekt kann der Transferpfad gerade sein. D. h., der Transferpfad kann bevorzugt gerade verlaufen bzw. einen linearen Verlauf aufweisen.

Zudem oder alternativ kann der Transferpfad ohne Richtungswechsel verlaufen. D. h., der Transferpfad kann bevorzugt entlang der kürzesten Verbindung zwischen der Ausgangsposition und der Freigabeposition verlaufen.

Zudem oder alternativ kann das Bewegen des Probenträgers mit der biologischen Probe von der Ausgangsposition in die Freigabeposition ausschließlich eine Translationsbewegung umfassen. D. h., das Bewegen des Probenträgers mit der biologischen Probe soll vorzugsweise keine Rotationsbewegung des Probenträgers mit der biologischen Probe umfassen. Auf vorteilhafte Weise kann dadurch insgesamt eine möglichst einfache und schnelle Transferbewegung erreicht werden.

Gemäß einem weiteren Aspekt kann der Transferpfad zur Trägerachse um einen Neigungswinkel geneigt verlaufen, wobei der Neigungswinkel ein Winkel zwischen 5° und 45°, vorzugsweise zwischen 10° und 30°, besonders bevorzugt zwischen 10° und 20° sein kann. Als Neigungswinkel kann dabei bevorzugt der, vorzugsweise spitze, Winkel verstanden werden, den die Trägerachse des Probenträgers mit dessen Bewegungsrichtung einschließt.

Zudem oder alternativ kann der Transferpfad zur Trägerachse um einen Neigungswinkel geneigt verlaufen, wobei der Neigungswinkel größer als ein Grenzwinkel βₘᵢₙ sein kann. D. h., der Transferpfad kann bevorzugt zumindest um den βₘᵢₙ geneigt zur Trägerachse verlaufen. Vorzugsweise ist dieser Grenzwinkel βₘᵢₙ dabei durch βₘᵢₙ = arctan((D/2-M+N)/L) bestimmt. Hierbei bezeichnet D eine laterale Ausdehnung (z. B. den Durchmesser) des Sockels, M einen vorgegebenen maximalen Abstand zwischen dem Flüssigkeitsdispenser und der biologischen Probe (z. B. 1 mm), N einen vorgegebenen Sicherheitsabstand zwischen dem Flüssigkeitsdispenser und dem Sockel und L eine Länge des Stifts. Hierbei kann M und/oder N ggf. auch den Wert 0 annehmen. Auf vorteilhafte Weise kann dadurch insgesamt ein kollisionsfreies Transferieren des Probenträgers mit der biologischen Probe sichergestellt werden.

Nach einem weiteren Aspekt kann die Trägerachse in der Ausgangsposition entlang der Schwerkraftrichtung orientiert sein. Beispielsweise kann das zweite Ende des Stifts des Probenträgers im Wesentlichen vertikal nach unten weisen und/oder die Probe unterhalb des Sockels angeordnet sein. Auf vorteilhafte Weise kann dadurch erreicht werden, dass der Probenhalter mit der Probe voran in das verflüssigte Gas eintaucht bzw. ggf. zunächst nur ein Teil des Stifts mit der Probe in das verflüssigte Gas eintauscht, um dadurch starke Turbulenzen im verflüssigten Gas während des Vitrifizierens möglichst zu vermeiden.

Gemäß einem weiteren Aspekt kann der Transferpfad derart verlaufen, dass sich der Probenträger und/oder die Transfereinrichtung beim Bewegen von der Ausgangsposition in die Freigabeposition kollisionsfrei am Flüssigkeitsdispenser vorbei bewegen. D. h., beim Bewegen von der Ausgangsposition in die Freigabeposition soll es bevorzugt zu keinem mechanischen Kontakt zwischen dem Probenträger und dem Flüssigkeitsdispenser bzw. der Transfereinrichtung und dem Flüssigkeitsdispenser kommen.

Nach einem weiteren Aspekt kann zwischen dem Zuführen der Flüssigkeit in der Ausgangsposition und dem Bewegen des Probenträgers mit der biologischen Probe von der Ausgangsposition in die Freigabeposition eine, vorzugsweise einstellbare, vorbestimmte Zeitspanne gewartet werden. D. h., das Verfahren kann eine, vorzugsweise einstellbare, Zeitverzögerung zwischen dem Zuführen der Flüssigkeit und dem Starten des Bewegens des Probenträgers mit der biologischen Probe umfassen. Über die Zeitverzögerung bzw. die vorbestimmte Zeitspanne kann auf vorteilhafte Weise der Zustand, in dem die biologische Probe nach dem Starten einer Reaktion (durch Flüssigkeitszugabe) letztlich vitrifiziert wird, eingestellt werden. Besonders bevorzugt ist hierbei vorgesehen, dass das Verfahren für mehrere Proben mit unterschiedlichen (z. B. ansteigenden) Zeitverzögerungen durchgeführt wird. Auf vorteilhafte Weise kann dadurch ein Probensatz für Synchrotronstrahlungsquellen bereitgestellt werden, aus welchem Erkenntnisse über das zeitliche Verhalten der Reaktion bzw. die zeitliche Veränderung der Struktur der Proben gewonnen werden können.

Zudem oder alternativ kann zwischen dem Zuführen der Flüssigkeit in der Ausgangsposition und dem Bewegen des Probenträgers mit der biologischen Probe von der Ausgangsposition in die Freigabeposition eine Lage des Flüssigkeitsdispensers nicht verändert werden. D. h., bevorzugt soll eine Position und Orientierung des Flüssigkeitsdispensers nach dem Zuführen der Flüssigkeit nicht variiert werden, zumindest bis sich der Probenträger mit der biologischen Probe in der Freigabeposition befindet. Insbesondere soll vorzugsweise somit kein Zurückziehen des Flüssigkeitsdispensers vor dem Transfervorgang erfolgen. Auf vorteilhafte Weise kann dadurch ein möglichst schneller Probentransfer nach dem Befeuchten sichergestellt werden. In diesem Zusammenhang sei jedoch erwähnt, dass der Flüssigkeitsdispenser, z. B. um ein Herausnehmen der vitrifizierten Proben zu erleichtern, grundsätzlich bewegbar (z. B. verschiebbar) ausgebildet sein kann. Allerdings während des vorgenannten Zeitraums kein entsprechendes Bewegen des Flüssigkeitsdispensers erfolgt.

Gemäß einem weiteren Aspekt kann das Verfahren ferner ein optisches Anregen der biologischen Probe in der Ausgangsposition mit zumindest einem Lichtimpuls mittels einer Bestrahlungseinrichtung umfassen. D. h., die biologische Probe kann bevorzugt mit einem Lichtimpuls bestrahlt werden, der von einer Bestrahlungseinrichtung (z. B. einem Laser) generiert wird. Neben dem Befeuchten der Probe kann somit auf vorteilhafte Weise auch durch eine entsprechende optische Anregung eine (z. B. chemische) Reaktion in der Probe induziert und/oder beeinflusst werden.

Nach einem weiteren Aspekt kann das Verfahren ferner ein Bereitstellen einer vorbestimmten Feuchte und/oder Temperatur in einer Umgebung der Probe in der Ausgangsposition mittels einer Konditioniereinrichtung umfassen. Auf vorteilhafte Weise kann dadurch die Probe vor einem Austrocknen geschützt werden und/oder eine definierte Temperatur eingestellt werden.

Gemäß einem weiteren Aspekt kann das Verfahren ferner ein Freigeben des Probenträgers mit der biologischen Probe aus der Transfereinrichtung in der Freigabeposition mittels einer Auslöseeinrichtung der Transfereinrichtung umfassen. D. h., bevorzugt kann die Auslöseeinrichtung dazu ausgebildet sein, eine form- und/oder kraftschlüssige Verbindung zwischen dem Probenträger mit der biologischen Probe und der Transfereinrichtung zu lösen. Vorzugsweise soll nach dem Freigeben der Probenträger mit der biologischen Probe somit aus der Transfereinrichtung entnehmbar sein. In einer bevorzugten Ausführungsform ist vorgesehen, dass das Freigeben des Probenträgers aus der Transfereinrichtung in der Freigabeposition mittels einer Auslöseeinrichtung der Transfereinrichtung so erfolgt, dass der Probenträger schwerkraftvermittelt aus der Transfereinrichtung fallen kann. Auf vorteilhafte Weise kann dadurch ein Ausgeben des Probenträger mit der biologischen Probe aus der Transfereinrichtung realisiert werden, welches kein äußeres Eingreifen nötig macht.

Nach einem zweiten unabhängigen Aspekt der Erfindung wird ein weiteres Verfahren bereitgestellt. Bevorzugt dient auch das weitere Verfahren zum, vorzugsweise automatisierten, Vitrifizieren einer biologischen Probe (z. B. einem Proteinkristall). Wie vorstehend bereits im Detail im Zusammenhang mit dem ersten unabhängigen Aspekt ausgeführt wurde, umfasst auch das weitere Verfahren gemäß dem zweiten unabhängigen Aspekt ein Positionieren eines Probenträgers mit der biologischen Probe mittels einer Transfereinrichtung in einer ersten Position, welche auch als "Ausgangsposition" bezeichnet werden kann. Auch hier ist vorgesehen, dass der Probenträger einen Sockel und einen (z. B. länglichen) Stift aufweist, wobei der Stift vom Sockel entlang einer Trägerachse absteht. Beispielsweise der Stift kann ein erstes Ende aufweisen, das am Sockel befestigt (z. B. eingesteckt) ist, und ein, dem ersten Ende entgegengesetztes, zweites Ende aufweisen, das frei ist. Der Probenträger kann somit z. B. ein nach dem SPINE-Standard ausgebildeter Probenträger sein. Weiterhin ist vorgesehen, dass die biologische Probe am Stift beabstandet vom Sockel (z. B. mittels eines Netzchens oder einer mikroskopisch kleinen Schleife) befestigt ist. D. h., die biologische Probe kann in einem vorbestimmten Abstand vom Sockel am Stift (z.B. an dessen zweiten Ende) gehaltert sein.

Auch das weitere Verfahren umfasst ferner ein Zuführen einer Flüssigkeit (z. B. Wasser oder eine wässrige Lösung einer Wirksubstanz) zu der biologischen Probe in der Ausgangsposition mittels eines Flüssigkeitsdispensers. Dieser kann dabei wie vorstehend im Zusammenhang mit dem ersten unabhängigen Aspekt beschrieben wurde, ausgebildet sein.

Das weitere Verfahren umfasst ferner ein Bewegen des Probenträgers mit der biologischen Probe mittels der Transfereinrichtung entlang eines vorbestimmten Transferpfads von der ersten Position bzw. Ausgangsposition in eine zweite Position, die im Folgenden als "Freigabeposition" bezeichnet werden kann. Auch hier ist vorgesehen, dass die biologische Probe in der Freigabeposition in oder angrenzend zu einem verflüssigten Gas (z. B. flüssigem Stickstoff oder flüssigem Ethan) angeordnet ist. Entsprechend kann auch hier in der Freigabeposition ein Einfrieren bzw. Schockgefrieren der (befeuchteten) biologischen Probe erfolgen. Dies kann z. B. wiederum dadurch erfolgen, dass die biologische Probe in der Freigabeposition bereits vom verflüssigten Gas umgeben ist oder dass die biologische Probe bzw. der Probenträger in der Freigabeposition aus der Transfereinrichtung ausgelöst bzw. freigegeben wird und schwerkraftvermittelt in das verflüssigte Gas fällt.

Während gemäß dem ersten unabhängigen Aspekt der Transferpfad geneigt zur Trägerachse verläuft, ist in der Alternative gemäß dem zweiten unabhängigen Aspekt vorgesehen, dass der Transferpfad entlang eines Kreisbogens verläuft. D. h., bevorzugt vollführt der Probenträger mit der biologischen Probe eine Teilkreisbewegung, während er sich von der Ausgangsposition in die Freigabeposition bewegt. Beispielsweise kann der Transferpfad in einer besonders bevorzugten Ausführungsform z. B. einen Viertelkreisbogen beschreiben. Auf vorteilhafte Weise kann dadurch sichergestellt werden, dass der Probenträger beim Transferieren nicht mit dem Flüssigkeitsdispenser kollidiert. Somit kann auf vorteilhafte Weise ein möglichst rasches Vitrifizieren der Proben erreicht werden, da keine Zeit für ein Zurückziehen des Flüssigkeitsdispensers verwendet werden muss. Ein weiterer Vorteil der vorgenannten kontrollierten Bewegung ist zudem, dass so in kurzer Zeit eine hohe Anzahl von Proben (z. B. mit unterschiedlichen Zeitverzögerungen und damit Reaktionszuständen) vitrifiziert werden können und damit ein Probensatz für Synchrotronstrahlungsquellen bereitgestellt werden kann, welcher zeitaufgelöste Strukturuntersuchungen ermöglicht.

Nach einem ersten Aspekt kann der Transferpfad im Wesentlichen entlang eines Viertelkreisbogens verlaufen. D. h., bevorzugt kann die Trägerachse in der Freigabeposition um 90° gedreht zur Trägerachse in der Ausgangsposition orientiert sein.

Zudem oder alternativ kann Bewegen des Probenträgers mit der biologischen Probe von der Ausgangsposition in die Freigabeposition ausschließlich eine Rotationsbewegung umfassen. D. h., das Bewegen des Probenträgers mit der biologischen Probe soll vorzugsweise keine Translationsbewegung des Probenträgers mit der biologischen Probe umfassen. Auf vorteilhafte Weise kann dadurch insgesamt eine möglichst einfache und rasche Transferbewegung erreicht werden.

Gemäß einem weiteren Aspekt kann die Trägerachse in der Ausgangsposition im Wesentlichen senkrecht zur Schwerkraftrichtung orientiert sein. Beispielsweise kann der Stift bzw. die Trägerachse des Probenträgers in der Ausgangsposition im Wesentlichen horizontal angeordnet sein.

Nach einem weiteren Aspekt kann das weitere Verfahren ferner - außer in den Fällen eindeutiger Unvereinbarkeit - die optionalen Merkmale umfassen, die vorstehend im Zusammenhang mit dem Verfahren gemäß dem ersten unabhängigen Aspekt ausgeführt wurden. Mit anderen Worten sollen die in diesem Dokument im Zusammenhang mit dem ersten unabhängigen Aspekt beschriebenen Merkmale - soweit übertragbar - auch im Zusammenhang mit dem zweiten unabhängigen Aspekt offenbart und beanspruchbar sein. Entsprechendes soll auch umgekehrt gelten. Insbesondere kann somit auch beim weiteren Verfahren ein optisches Anregen der biologischen Probe, ein Bereitstellen einer vorbestimmten Feuchte und/oder Temperatur in einer Umgebung der Probe und/oder ein Freigeben des Probenträgers aus der Transfereinrichtung in der Freigabeposition vorgesehen sein.

Weiterhin betrifft die Erfindung auch eine Vitrifizierungsvorrichtung, die zur Durchführung eines der in diesem Dokument beschriebenen Verfahren ausgebildet ist. Auch hier sollen die in diesem Dokument im Zusammenhang mit den Verfahren beschriebenen Merkmale auch im Zusammenhang mit der Vitrifizierungsvorrichtung offenbart und beanspruchbar sein. Entsprechendes soll auch umgekehrt gelten.

Die Vitrifizierungsvorrichtung kann dabei einen Behälter zur Aufnahme des verflüssigten Gases aufweisen. Der Behälter kann bspw. zur Aufnahme von flüssigen Stickstoff, Ethan und/oder Methan ausgebildet sein. Beispielsweise kann es sich bei dem Behälter um eine wärmeisolierende Wanne und/oder ein Dewargefäß handeln.

Ferner kann die Vitrifizierungsvorrichtung eine Transfereinrichtung mit einer Halterung zur Aufnahme des Probenträgers mit der biologischen Probe aufweisen. Die Halterung kann bspw. eingerichtet sein, den Probenträger mit der biologischen Probe form- und/oder kraftschlüssig an der Transfereinrichtung zu fixieren. Beispielsweise kann die Halterung dazu einen Elektromagneten und/oder eine Klemmvorrichtung umfassen. Weiterhin kann die Transfereinrichtung ausgebildet ist, den in der Halterung aufgenommenen Probenträger mit der biologischen Probe in der Ausgangsposition zu positionieren und entlang des vorbestimmten Transferpfads von der Ausgangsposition in die Freigabeposition zu bewegen. Zudem oder alternativ kann die Transfereinrichtung auch ausgebildet ist, mittels der Halterung den Probenträger mit der biologischen Probe in der Ausgangsposition zu positionieren und entlang des vorbestimmten Transferpfads von der Ausgangsposition in die Freigabeposition zu bewegen. Hierzu kann die Transfereinrichtung bspw. über (z. B. pneumatische und/oder hydraulische) Aktoren und/oder Führungen verfügen, um eine entsprechende Bewegung des Probenträgers mit der biologischen Probe zu ermöglichen. Lediglich beispielhaft kann im Fall, dass der Transferpfad linear verläuft, das Bewegen des Probenträgers mit der biologischen Probe bspw. mittels einer Linearverstellung erfolgen. Im Fall, dass der Transferpfad entlang eines Kreisbogens verläuft, kann das Bewegen des Probenträgers mit der biologischen Probe bspw. mittels eines Servomotors erfolgen.

Ferner kann die Vitrifizierungsvorrichtung auch einen Flüssigkeitsdispenser zum Zuführen der Flüssigkeit zu der biologischen Probe in der Ausgangsposition umfassen. Der Flüssigkeitsdispenser kann hierbei ausgebildet sein, die entsprechende Flüssigkeit tröpfchenweise (z. B. mit Tröpfchengrößen von etwa 50 µm) abzugeben. Bevorzugt ist der Flüssigkeitsdispenser hierbei ausgebildet, eine Tropfenfolge mit einer Erzeugungsrate von 1-2 kHz abzugeben, um dadurch auf vorteilhafte Weise möglichst kontrolliert einen Flüssigkeitsfilm auf der biologischen Probe zu erzeugen. In einer Ausführungsform kann die Vitrifizierungsvorrichtung auch mehrere Flüssigkeitsdispenser zum Zuführen der Flüssigkeit zu der biologischen Probe in der Ausgangsposition umfassen.

Weiterhin kann die Vitrifizierungsvorrichtung zumindest eine der folgenden optionalen Komponenten umfassen: eine Bestrahlungseinrichtung, eine Konditioniereinrichtung, eine Auslöseeinrichtung und eine Lagereinrichtung.

Die Bestrahlungseinrichtung (z. B. ein Laser) kann hierbei angeordnet sein, die biologische Probe in der Ausgangsposition mittels zumindest eines Lichtimpulses optisch anzuregen. D. h., die biologische Probe soll sich in der Ausgangsposition bevorzugt im Strahlengang der Bestrahlungseinrichtung befinden. Auf vorteilhafte Weise kann hierdurch eine (z. B. chemische) Reaktion in der Probe angeregt und/oder beeinflusst werden.

Die Konditioniereinrichtung kann zur Bereitstellung einer vorbestimmten Feuchte und/oder Temperatur in einer Umgebung der biologischen Probe in der Ausgangsposition eingerichtet sein. Beispielsweise kann die Konditioniereinrichtung ausgebildet sein, einen Wassernebel und/oder temperierte Luft in der Umgebung der biologischen Probe in der Ausgangsposition zu erzeugen.

Die Auslöseeinrichtung kann bevorzugt dazu ausgebildet sein, eine form- und/oder kraftschlüssige Verbindung zwischen dem Probenträger mit der biologischen Probe und der Transfereinrichtung zu lösen. Mittels der Auslöseeinrichtung kann somit der Probenträger mit der biologischen Probe für eine Entnahme des Probenträger aus der Transfereinrichtung und/oder der Halterung freigebbar sein.

Die Lagereinrichtung ist vorzugsweise in dem Behälter zur Aufnahme des verflüssigten Gases angeordnet. Weiterhin kann die Lagereinrichtung (z. B. in Form eines drehbaren Magazins) zumindest eine Aufnahme aufweisen, in die der Probenträger mit der biologischen Probe aufnehmbar ist. Bevorzugt ist die zumindest eine Aufnahme dabei derart ausgebildet, dass die in der zumindest einen Aufnahme aufgenommene Probe nicht in direktem Kontakt mit der Lagereinrichtung steht. In einer besonders bevorzugten Ausführungsform ist die Lagereinrichtung ferner derart angeordnet, dass, wenn sich der Probenträger mit der biologischen Probe in der Freigabeposition befindet, die zumindest eine Aufnahme unterhalb des Probenträgers mit der biologischen Probe angeordnet ist. Auf vorteilhafte Weise kann dadurch der Probenträger mit der biologischen Probe schwerkraftvermittelt und damit quasi selbsttätig nach Freigeben aus der Halterung der Transfereinrichtung mittels der Auslöseeinrichtung in die zumindest eine Aufnahme der Lagereinrichtung fallen.

Die zuvor beschriebenen Aspekte und Merkmale der Erfindung sind miteinander kombinierbar. Weitere Einzelheiten und Vorteile der Erfindung werden im Folgenden unter Bezugnahme auf die beigefügten Zeichnungen beschrieben.

### Figurenbeschreibung

- Figur 1:: ein Flussdiagramm eines Verfahrens zum Vitrifizieren einer biologischen Probe gemäß einer Ausführungsform;
- Figur 2:: eine schematische Darstellung einer Vitrifizierungsvorrichtung gemäß einer Ausführungsform, wobei sich der Probenträger mit der biologischen Probe in der Ausgangsposition befindet;
- Figur 3:: eine schematische Darstellung der in Figur 2 gezeigten Vitrifizierungsvorrichtung, wobei sich der Probenträger mit der biologischen Probe in der Freigabeposition befindet;
- Figur 4:: eine weitere schematische Darstellung der in Figur 2 und 3 gezeigten Vitrifizierungsvorrichtung;
- Figur 5:: eine schematische Darstellung der geometrischen Zusammenhänge zwischen dem Probenhalter und dem Flüssigkeitsdispenser zur Festlegung eines Grenzwinkels für einen Neigungswinkel des Transferpads gemäß einer Ausführungsform; und
- Figur 6:: eine schematische Darstellung einer Vitrifizierungsvorrichtung gemäß einer weiteren Ausführungsform, wobei sich der Probenträger mit der biologischen Probe in der Ausgangsposition befindet.

Gleiche oder funktional äquivalente Elemente sind in allen Figuren mit denselben Bezugszeichen beschrieben und zum Teil nicht gesondert beschrieben.

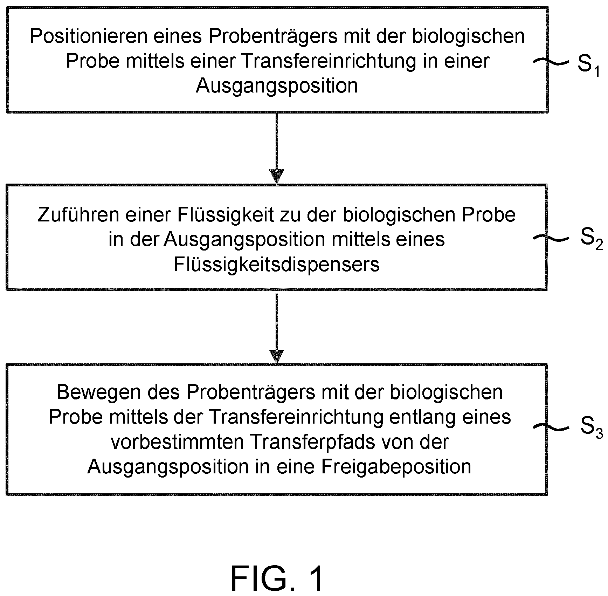

Figur 1 zeigt ein Flussdiagramm eines Verfahrens zum Vitrifizieren einer biologischen Probe 1. Bei der biologischen Probe 1 kann es sich beispielsweise um eine organische Substanz, wie z. B. Biomoleküle, handeln. Im Schritt S₁ erfolgt ein Positionieren eines Probenträgers 2 mit der biologischen Probe 1 mittels einer Transfereinrichtung 12 in einer als Ausgangsposition bezeichneten ersten Position P₁. D. h., bevorzugt wird der Probenträger 2 mit der biologischen Probe 1 in eine vorbestimmte Position (= Ausgangsposition Pi) gebracht. Die Ausgangsposition P₁ kann dabei vorzugsweise einen Ort und/oder eine Orientierung des Probenträgers 2 mit der biologischen Probe 1 im dreidimensionalen Raum beschreiben. Der Probenträger 2 soll hierbei bevorzugt einen Sockel 2a und einen, vom Sockel 2a entlang einer Trägerachse A abstehenden, Stift 2b aufweisen. Beispielsweise kann der Probenträger 2 ein nach dem SPINE-Standard ausgebildeter Probenträger 2 sein. Bevorzugt ist vorgesehen, dass die biologische Probe 1 beabstandet vom Sockel 2a (z. B. mittels eines Netzchens oder einer mikroskopischen Schleife) am Stift 2b befestigt ist. Beispielsweise kann ein Abstand zwischen der biologischen Probe 1 und einem, entgegensetzten zum Stift 2b orientierten, Boden des Sockels 2a 22 mm sein.

Im Schritt S₂ erfolgt ein Zuführen einer Flüssigkeit (z. B. Wasser oder eine flüssige Lösung einer Wirksubstanz) zu der biologischen Probe 1 in der Ausgangsposition P₁ mittels eines Flüssigkeitsdispensers 13. Bevorzugt dient das Zuführen der Flüssigkeit hierbei zum Starten einer, z. B. chemischen und/oder enzymatischen, Reaktion in der biologischen Probe 1. Der Flüssigkeitsdispenser 13 kann dabei ausgebildet sein, die Flüssigkeit tröpfchenweise, z. B. mit einer durchschnittlichen Tröpfchengröße von etwa 50 µm und/oder einer Tröpfchen-Erzeugungsrate von etwa 1-2 kHz, der biologischen Probe 1 zuzuführen. Der Flüssigkeitsdispenser 13 kann ferner zum Zuführen der Flüssigkeit wenige Millimeter, vorzugsweise < 1 mm, von der biologischen Probe 1 beabstandet sein. Entsprechend kann das Verfahren auch ein Positionieren des Flüssigkeitsdispensers 13 umfassen, derart, dass der Flüssigkeitsdispenser 13, insbesondere eine Flüssigkeitsaustrittsöffnung des Flüssigkeitsdispensers 13, von der biologischen Probe 1 in der Ausgangsposition P₁ weniger als 2 mm, vorzugsweise weniger als 1 mm, beabstandet ist.

Im Schritt S₃ erfolgt ein Bewegen des Probenträgers 2 mit der biologischen Probe 1 mittels der Transfereinrichtung 12 entlang eines vorbestimmten Transferpfads T von der ersten Position bzw. Ausgangsposition P₁ in eine als Freigabeposition bezeichnete zweite Position P₂. Das Bewegen des Probenträgers 2 mit der biologischen Probe 1 kann hierbei auch als Transferieren bezeichnet werden. Der Ausdruck Transferpfad T kann dabei vorzugsweise den Weg der Bewegung des Probenträgers 2 mit der biologischen Probe 1 von der Ausgangsposition P₁ zur Freigabeposition P₂ bezeichnen. Beispielsweise kann der Transferpfad T der Weg der Bewegung des Schwerpunkts des Probenträgers 2 und/oder der Probe 1 sein. Hierbei ist vorgesehen, dass die biologische Probe 1 in der Freigabeposition P₂ in oder angrenzend zu einem verflüssigten Gas 3 angeordnet ist. Als verflüssigtes Gas 3, welches auch als Flüssiggas bezeichnet werden kann, soll hierbei bevorzugt ein durch Kühlung und/oder Kompression verflüssigtes Gas 3 verstanden werden, das bei Normaldruck aufgrund der Verdampfungsenthalpie kalt und flüssig bleibt. Beispielsweise kann es sich bei dem verflüssigten Gas 3 um flüssigen Stickstoff, flüssiges Methan und/oder flüssiges Ethan handeln. Je nachdem, ob die Freigabeposition P₂ im verflüssigten Gas 3 oder angrenzend dazu liegt, kann in der Freigabeposition P₂ entweder sofort ein Vitrifizieren der biologischen Probe 1 erfolgen oder erst nach einem entsprechenden Eintauchen der biologischen Probe 1 in das verflüssigte Gas 3 (z. B. nach einem Freigeben des Probenträgers 2 aus der Transfereinrichtung 12 und einem darauffolgenden freien Fall in das verflüssigte Gas 3). Auf vorteilhafte Weise kann so der aktuelle Zustand der biologischen Probe 1 (z. B. ein durch die Flüssigkeitszugabe erzeugtes Reaktionsintermediat) für nachfolgende Untersuchungen (z. B. an einer Synchrotronstrahlungsquelle) konserviert werden.

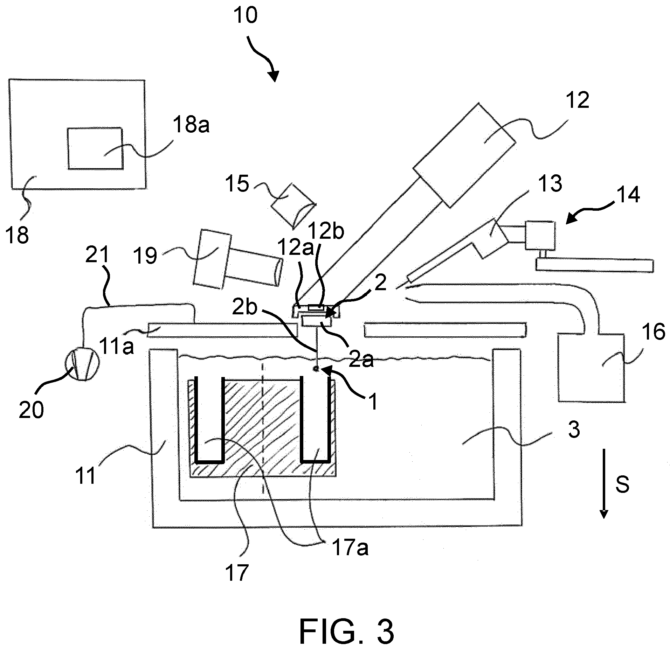

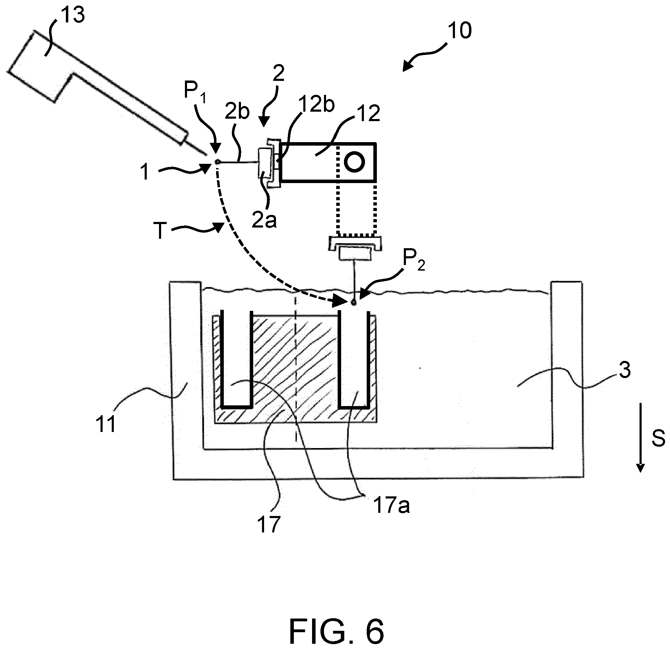

Das entsprechende Verfahren zeichnet sich dadurch aus, dass der vorgenannte Transferpfad T zur Trägerachse A geneigt verläuft (Alternative 1) oder dass der Transferpfad T entlang eines Kreisbogens verläuft (Alternative 2), was im Zusammenhang mit den Figuren 3 und 6 noch eingehender beschrieben werden wird.

Weiterhin sieht eine bevorzugte Ausführungsform vor, dass das vorstehend beschriebene Verfahren für mehrere biologische Proben 1 (z. B. parallel oder nacheinander) durchgeführt wird, wobei sich die jeweiligen Verfahrensdurchläufe jeweils in einer Zeitverzögerung zwischen dem Zuführen der Flüssigkeit und dem Starten des Bewegens des Probenträgers 2 mit der biologischen Probe 1 unterscheiden. Lediglich beispielhafte können so zehn biologische Proben 1 mit zehn verschiedenen (z. B. ansteigenden) Zeitverzögerungen vitrifiziert werden, um so durch nachfolgende Strukturuntersuchungen der biologischen Proben 1 an einer Synchrotronstrahlungsquelle auf vorteilhafte Weise Erkenntnisse über das zeitliche Verhalten der Reaktion bzw. die zeitliche Veränderung der Struktur der biologischen Proben 1 gewinnen zu können.

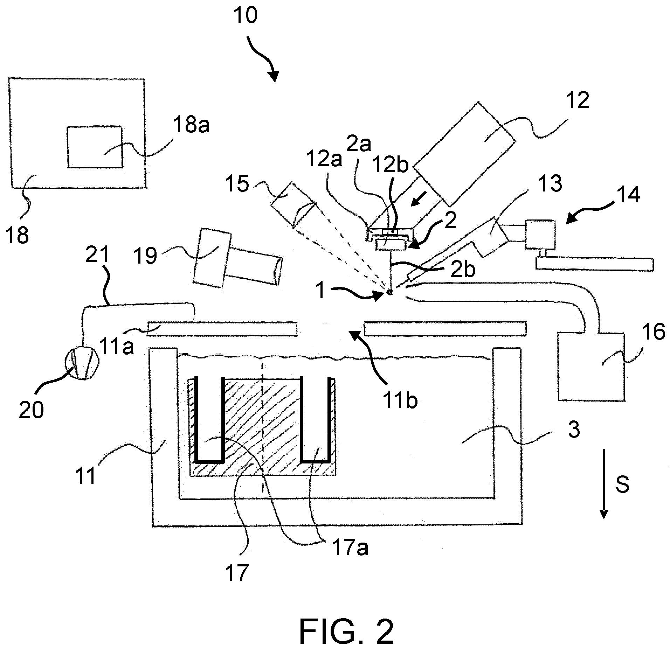

Die Figuren 2 bis 4 zeigen verschiedene schematische Darstellung einer Vitrifizierungsvorrichtung 10 gemäß einer Ausführungsform. Die Vitrifizierungsvorrichtung 10 ist hierbei zur Durchführung eines der Verfahren, wie in diesem Dokument (z. B. im Zusammenhang mit Figur 1) beschrieben, ausgebildet.

Die Vitrifizierungsvorrichtung 10 kann dazu einen Behälter 11 zur Aufnahme des verflüssigten Gases 3 aufweisen. Beispielsweise kann der Behälter 11 zur Aufnahme von flüssigem Stickstoff und/oder flüssigem Methan ausgebildet sein. Der Behälter 11 kann z. B. wannenförmig sein. Der Behälter 11 kann bis zu einem vergebenen Soll-Füllstand mit dem verflüssigten Gas 3 befüllbar sein. Der Behälter 11 kann einen Deckel 11a mit zumindest einer Ausnehmung 11b (z. B. einer Aussparung) aufweisen. Der Deckel 11a mit der mit zumindest einen Ausnehmung 11b kann den Behälter 11 von oben verschließen, wobei über die zumindest eine Ausnehmung 11b bevorzugt ein Zugang von außen ins Innere des Behälters 11 möglich sein soll. Vorzugsweise ist die zumindest einer Ausnehmung 11b ausgebildet, dass über diese ein Einführen des Probenträgers 2 bzw. der biologischen Probe 1 ins Innere des Behälters 11 möglich ist. Der Deckel 11a kann dazu dienen, in dem Behälter 11 aufgenommenes verflüssigtes Gas 3 von der Umgebung thermisch zu isolieren. D. h., durch den Deckel 11a kann ein direkter Kontakt zwischen dem verflüssigten Gas 3 und der Umgebungsluft reduziert werden. Weiterhin kann die Vitrifizierungsvorrichtung 10 eine Pumpe 20 (z. B. eine Membranpumpe) umfassen. Die Pumpe 20 kann dazu dienen, oberhalb des verflüssigten Gases 3 entstehende (kalte) Gase abzusaugen, um dadurch ein Entstehen von Nebel aus der Restfeuchtigkeit der Umgebungsluft zu vermindern. Hierzu kann die Pumpe 20, z. B. über eine den Deckel 11a durchdringende Leitungsverbindung 21 mit einem zwischen dem Deckel 11a und dem verflüssigten Gas 3 bzw. dem Soll-Füllstand angeordneten Bereich des Behälters 11 in Fluidverbindung stehen.

Die Vitrifizierungsvorrichtung 10 kann ferner eine Transfereinrichtung 12 mit einer Halterung 12a zur Aufnahme des Probenträgers 2 mit der biologischen Probe 1 aufweisen. Die Halterung 12a kann bspw. eingerichtet sein, den Probenträger 2 mit der biologischen Probe 1 form- und/oder kraftschlüssig an der Transfereinrichtung 12 zu fixieren. Beispielsweise kann die Halterung 12a dazu einen Elektromagneten und/oder eine Klemmvorrichtung umfassen. Die Halterung 12a kann zumindest abschnittsweise formangepasst an den Probenträger 2 sein. Beispielsweise kann eine Innenkontur der Halterung 12a zumindest abschnittsweise formangepasst zu einer Außenkontur des Sockels 2a des Probenträgers 2 ausgebildet sein. Weiterhin kann die Transfereinrichtung 12 eine Auslöseeinrichtung 12b aufweisen. Die Auslöseeinrichtung 12b kann dazu ausgebildet sein, eine form-und/oder kraftschlüssige Verbindung zwischen dem Probenträger 2 mit der biologischen Probe 1 und der Transfereinrichtung 12 zu lösen. Mittels der Auslöseeinrichtung 12b kann somit der Probenträger 2 mit der biologischen Probe 1 für eine Entnahme des Probenträger 2 mit der biologischen Probe 1 aus der Transfereinrichtung 12 bzw. der Halterung 12a freigebbar sein.

Die Transfereinrichtung 12 kann ferner ausgebildet sein, den in der Halterung 12a aufgenommenen Probenträger 2, vorzugsweise ein nach dem SPINE-Standard ausgebildeter Probenträger 2, mit der biologischen Probe 1 in der Ausgangsposition P₁ zu positionieren (vgl. Figur 2). D. h., die Transfereinrichtung 12 kann dazu ausgebildet sein, mittels der Halterung 12a den Probenträger 2 mit der biologischen Probe 1 an einem vorbestimmten Ort und/oder in einer vorbestimmten Orientierung im dreidimensionalen Raum zu halten. Vorzugsweise befindet sich die Ausgangsposition P₁ oberhalb des Behälters 11. Weiterhin bevorzugt ist die Trägerachse A in der Ausgangsposition P₁ entlang der Schwerkraftrichtung S orientiert. Die Trägerachse A kann jedoch auch schräg zur Schwerkraftrichtung S orientiert sein. Lediglich beispielhaft kann die Halterung 12a eine nach unten orientierte Anlagefläche aufweisen, an der Sockel 2a des Probenträgers 2 im gehalterten Zustand anliegt, sodass die, am Stift 2b befestigte, biologische Probe 1 in der Ausgangsposition P₁ unterhalb des Sockels 2a angeordnet ist.

Weiterhin kann die Vitrifizierungsvorrichtung 10 zumindest einen Flüssigkeitsdispenser 13 zum Zuführen der Flüssigkeit zu der biologischen Probe 1 in der Ausgangsposition P₁ umfassen. Der zumindest eine Flüssigkeitsdispenser 13 kann ausgebildet sein, die entsprechende Flüssigkeit tröpfchenweise (z. B. mit Tröpfchengrößen von etwa 50 µm) abzugeben. Der zumindest eine Flüssigkeitsdispenser 13 kann ausgebildet, eine Tropfenfolge mit einer Erzeugungsrate von 1-2 kHz abzugeben, um dadurch auf vorteilhafte Weise möglichst kontrolliert einen Flüssigkeitsfilm auf der biologischen Probe 1 zu erzeugen. Beispielsweise kann der zumindest eine Flüssigkeitsdispenser 13 hierbei eine mit einem Flüssigkeitsreservoir (nicht dargestellt) fluidisch verbundene Kanüle mit einer Austrittsöffnung aufweisen. Die Austrittsöffnung kann in einem Abstand von wenigen Millimetern, vorzugsweise in einem Abstand < 1 mm, von der biologischen Probe 1 positioniert werden oder sein. Hierzu kann die Vitrifizierungsvorrichtung 10 zumindest eine Verstelleinrichtung 14 (z. B. eine Linearverstellung) umfassen, mittels derer der zumindest eine Flüssigkeitsdispenser 13 bewegbar ist. Beispielsweise kann der zumindest eine Flüssigkeitsdispenser 13 bzw. die Austrittsöffnung mittels der zumindest einen Verstelleinrichtung 14 an die biologische Probe 1 heranfahrbar und/oder von der biologischen Probe 1 zurückziehbar sein. In einer bevorzugten Ausführung umfasst die zumindest eine Verstelleinrichtung 14 dazu zumindest zwei Verstelleinrichtungen 14 (nicht gesondert referenziert). Beispielsweise können die zumindest zwei Verstelleinrichtungen 14 eine Grob-Verstelleinrichtung umfassen, mittels derer der zumindest eine Flüssigkeitsdispenser 13 (z. B. um einen besseren Zugang zum Wechsel des Probenhalters 2 zu erhalten) über größere Distanzen bewegbar ist, und eine Fein-Verstelleinrichtung umfassen, mittels derer ein möglichst exaktes Positionieren des zumindest einen Flüssigkeitsdispensers 13 bzw. der Austrittsöffnung an der biologischen Probe 1 möglich ist. Durch das Zuführen der Flüssigkeit bzw. dem Befeuchten der biologischen Probe 1 mittels des zumindest einen Flüssigkeitsdispensers 13 kann bspw. eine chemische und/oder enzymatische Reaktion in der biologischen Probe 1 ausgelöst werden.

Um den zumindest einen Flüssigkeitsdispenser 13 möglichst exakt an der biologischen Probe 1 zu positionieren, kann die Vitrifizierungsvorrichtung 10 weiterhin zumindest eine Makrokamera 19 umfassen. Bevorzugt weist die zumindest eine Makrokamera 19 mehrere (z. B. zwei) Makrokameras 19 auf, welche vorzugsweise in unterschiedlichen Blickwinkel auf die biologischen Probe 1 gerichtet sind. Die zumindest eine Makrokamera 19 kann hierbei angeordnet sein, einen räumlichen Bereich um die biologische Probe 1 in der Ausgangsposition P₁ zu erfassen. Bevorzugt ist dabei eine der zumindest einen Makrokamera 19 senkrecht zu einer Bewegungsrichtung des zumindest einen Flüssigkeitsdispensers 13 orientiert angeordnet. Die Vitrifizierungsvorrichtung 10 kann ferner eine Ausgabeeinrichtung (z. B. einen Bildschirm) umfassen (nicht dargestellt), auf dem von der zumindest einen Makrokamera 19 erfasste Bilddaten ausgegeben werden.

Weiterhin kann die Vitrifizierungsvorrichtung 10 eine, vorzugsweise schaltbare, Bestrahlungseinrichtung 15 umfassen. Die Bestrahlungseinrichtung 15 (z. B. ein Laser) kann hierbei angeordnet sein, die biologische Probe 1 in der Ausgangsposition P₁ mittels zumindest eines Lichtimpulses optisch anzuregen. D. h., mittels der Bestrahlungseinrichtung 15 kann die biologische Probe 1 mit einem Lichtimpuls bestrahlt werden, der von einer Bestrahlungseinrichtung 15 generiert wird. Entsprechend kann sich die biologische Probe 1 in der Ausgangsposition P₁ bevorzugt im Strahlengang der Bestrahlungseinrichtung 15 (angedeutet durch die gestrichelte Linie) befinden. Zusätzlich oder alternativ zum Befeuchten der biologischen Probe 1 in der Ausgangsposition P₁ kann so ebenfalls auf vorteilhafte Weise eine Reaktion in der biologischen Probe 1 induziert und/oder beeinflusst werden.

Die Vitrifizierungsvorrichtung 10 kann ferner eine Konditioniereinrichtung 16 umfassen. Die Konditioniereinrichtung 16 kann zur Bereitstellung einer vorbestimmten Feuchte und/oder Temperatur in einer Umgebung der biologischen Probe 1 in der Ausgangsposition P₁ eingerichtet sein. Beispielsweise kann die Konditioniereinrichtung 16 ausgebildet sein, einen Wassernebel und/oder temperierte Luft in der Umgebung der biologischen Probe 1 in der Ausgangsposition P₁ zu erzeugen. D. h., mittels der Konditioniereinrichtung 16 kann eine kontrollierte Atmosphäre in der Nähe der biologischen Probe 1 bereitgestellt werden. Auf vorteilhafte Weise kann die biologische Probe 1 dadurch vor einem Austrocknen geschützt werden. Zudem oder alternativ können so auch möglichst konstante Reaktionsbedingungen bereitgestellt werden.

Nach einem entsprechenden Präparieren bzw. Modifizieren der biologischen Probe 1 in der Ausgangsposition P₁ kann diese sodann für nachfolgende Untersuchungen (z. B. an einer Synchrotronstrahlungsquelle) konserviert bzw. vitrifiziert werden. Hierzu kann die Transfereinrichtung 12 ausgebildet sein, den in der Halterung 12a aufgenommenen Probenträger 2 mit der biologischen Probe 1 entlang des vorbestimmten Transferpfads T von der Ausgangsposition P₁ in die Freigabeposition P₂ zu bewegen (vgl. Figur 2). D. h., die Transfereinrichtung 12 kann dazu ausgebildet sein, mittels der Halterung 12a den Probenträger 2 mit der biologischen Probe 1 entlang des vorbestimmten Transferpfads T von der Ausgangsposition P₁ in die Freigabeposition P₂ zu transferieren. In der Freigabeposition P₂ kann die biologische Probe 1 - wie dargestellt - in dem verflüssigten Gas 3 (z. B. flüssigem Stickstoff oder flüssigem Ethan) angeordnet sein. Entsprechend kann in der Freigabeposition P₂ unmittelbar ein Vitrifizieren bzw. Schockgefrieren der biologischen Probe 1 erfolgen. In einer alternativen Variante kann die biologische Probe 1 in der Freigabeposition P₂ zunächst auch noch oberhalb des verflüssigten Gases 3 bzw. oberhalb des Soll-Füllstands angeordnet sein (nicht dargestellt). Bevorzugt ist die biologische Probe 1 in der Freigabeposition P₂ dabei angrenzend zum verflüssigten Gas 3 bzw. dem Soll-Füllstand angeordnet. D. h. die biologische Probe 1 kann sich in der Freigabeposition P₂ in unmittelbarer räumlicher Nähe zum verflüssigten Gas 3 bzw. dem Soll-Füllstand befinden, z. B. weniger als 1 cm vom verflüssigten Gas 3 bzw. dem Soll-Füllstand entfernt. In dieser Ausführungsform kann das Vitrifizieren der biologischen Probe 1 nach einem Freigeben des Probenträgers 2 mit der biologischen Probe 1 aus der Transfereinrichtung 12 bzw. der Halterung 12a durch die Auslöseeinrichtung 12b und einem anschließenden schwerkraftvermittelten Fallen des Probenträgers 2 mit der biologischen Probe 1 in das verflüssigten Gases 3 erfolgen.

Bevorzugt ist hierbei vorgesehen, dass die Vitrifizierungsvorrichtung 10 eine Lagereinrichtung 17 umfasst. Die Lagereinrichtung 17 kann hierbei in dem Behälter 11 zur Aufnahme des verflüssigten Gases 3 angeordnet sein. Die Lagereinrichtung 17 kann beispielsweise in Form eines drehbaren Magazins ausgebildet sein. Die Lagereinrichtung 17 kann zumindest eine Aufnahme 17a aufweisen. Bevorzugt weist die zumindest eine Aufnahme 17a mehrere (z. B. zehn) Aufnahmen 17a auf. In der zumindest eine Aufnahme 17a kann die biologische Probe 1 aufnehmbar sein. Bevorzugt ist die zumindest eine Aufnahme 17a dabei derart ausgebildet, dass die in der zumindest einen Aufnahme 17a aufgenommene biologische Probe 1 nicht in direktem Kontakt mit der Lagereinrichtung 17 steht. Beispielsweise kann die zumindest eine Aufnahme 17a in Form eines zylinderförmigen und/oder phiolenförmigen Behältnisses ausgebildet sein. Vorzugsweise ist dabei ein Durchmesser des Behältnisses an einen Durchmesser des Sockels 2a des Probenträgers 2 derart angepasst, dass der Sockel 2a nur abschnittsweise in dem Behältnis aufnehmbar ist. Weiterhin bevorzugt ist die zumindest eine Aufnahme 17a mitsamt des Probenträgers 2 mit der biologischen Probe 1 auf der Lagereinrichtung 17 entnehmenbar. Ferner kann die Lagereinrichtung 17 derart angeordnet sein, dass, wenn sich der Probenträger 2 mit der biologischen Probe 1 in der Freigabeposition P₂ befindet, die zumindest eine Aufnahme 17a exakt unterhalb des Probenträgers 2 mit der biologischen Probe 1 angeordnet ist. Auf vorteilhafte Weise kann dadurch der Probenträger 2 mit der biologischen Probe 1 schwerkraftvermittelt und damit quasi selbsttätig nach einem Freigeben aus der Halterung 12a der Transfereinrichtung 12 mittels der Auslöseeinrichtung 12b in die zumindest eine Aufnahme 17a der Lagereinrichtung 17 fallen. In einer weiteren Variante kann auch vorgesehen sein, dass die Vitrifizierungsvorrichtung 10 lediglich eine entsprechende Befestigungsstelle zur Anbringung der Lagereinrichtung 17 aufweist, ohne dass die Lagereinrichtung 17 selbst zwingender Bestandteil der Vitrifizierungsvorrichtung 10 ist.

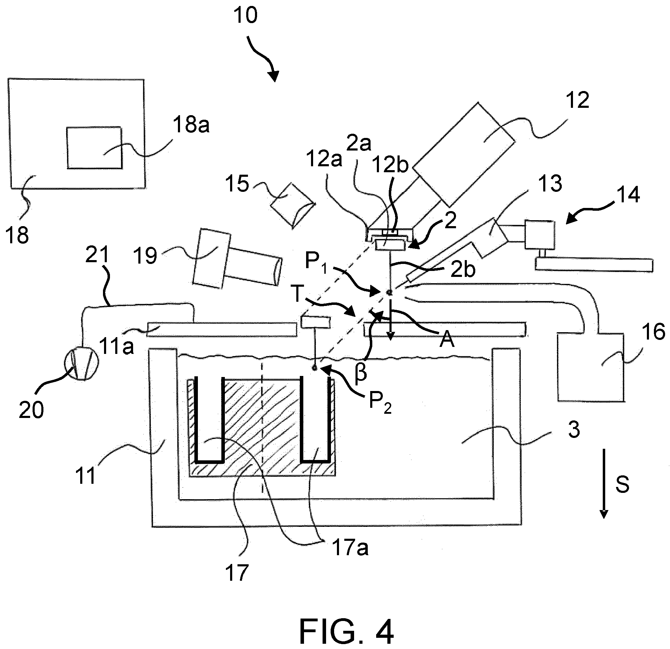

Nachdem vorstehend vorrangig die Konfiguration der Vitrifizierungsvorrichtung 10 in der Ausgangsposition P₁ (Figur 2) und in der Freigabeposition P₂ (Figur 3) diskutiert wurden, soll im Folgenden unter Bezugnahme auf Figur 4 nochmals auf den Transferpfad T bzw. die Transferbewegung eingegangen werden. So ist in dieser Ausführungsform vorgesehen, dass der Transferpfad T zur Trägerachse A geneigt verläuft. Wie vorstehend erwähnt, soll der Ausdruck Transferpfad T dabei vorzugsweise den Weg der Bewegung des Probenträgers 2 mit der biologischen Probe 1 von der Ausgangsposition P₁ zur Freigabeposition P₂ bezeichnen. Beispielsweise kann der Transferpfad T der Weg der Bewegung des Schwerpunkts der Probe 1 sein (vgl. gestrichelte Linie in Figur 4). Wie beispielhaft dargestellt ist, kann der Transferpfad T in einem Neigungswinkel β von 45° zur Trägerachse A verlaufen. Bevorzugt ist jedoch ein Neigungswinkel β zwischen 10° und 20° vorgesehen. Weiterhin soll der Transferpfad T in dieser Ausführungsform bevorzugt ohne Richtungswechsel verlaufen. Entsprechend kann das Bewegen des Probenträgers 2 mit der biologischen Probe 1 von der Ausgangsposition P₁ in die Freigabeposition P₂ hier beispielhaft ausschließlich eine Translationsbewegung umfassen. Weiterhin bevorzugt ist vorgesehen, dass eine Dauer der Transferbewegung einiger im Bereich einiger zehntel bis hundert Millisekunden (z. B. 50 ms) liegt.

Hierzu kann die Transfereinrichtung 12 bspw. über (z. B. pneumatische und/oder hydraulische) Aktoren und/oder Führungen verfügen, um eine entsprechende Bewegung des Probenträgers 2 mit der biologischen Probe 1 zu ermöglichen. Beispielsweise kann in der vorliegenden Ausführungsform die Transfereinrichtung 12 eine (z. B. pneumatische) Lineareinheit aufweist, die ausgebildet ist, die Halterung 12a bzw. den Probenträger 2 mit der biologischen Probe 1 entlang einer Geraden zu verfahren. In diesem Zusammenhang sei darauf hingewiesen, dass der Probenträger 2 mit der biologischen Probe 1 nicht zwingend Bestandteil der Vitrifizierungsvorrichtung 10 ist, sondern die Vitrifizierungsvorrichtung 10 lediglich dazu ausgebildet ist, eine entsprechende Bewegung der entsprechenden Komponenten entlang des Transferpfads T zu ermöglichen. In einer Variante kann die Vitrifizierungsvorrichtung 10 jedoch auch den Probenträger 2 mit der biologischen Probe 1 umfassen.

Bevorzugt ist weiterhin vorgesehen, dass mittels der Vitrifizierungsvorrichtung 10 eine Zeitverzögerung zwischen dem Zuführen der Flüssigkeit in der Ausgangsposition P₁ und dem Bewegen des Probenträgers 2 mit der biologischen Probe 1 von der Ausgangsposition P₁ in die Freigabeposition P₂ einstellbar ist. Über die vorgenannte Zeitverzögerung kann auf vorteilhafte Weise der Zustand, in dem die biologische Probe 1 nach dem Starten einer Reaktion (durch Flüssigkeitszugabe) letztlich vitrifiziert wird, veränderbar sein. Dies ist besonders vorteilhaft, falls das Verfahren für mehrere (gleiche) biologische Proben 1 mit unterschiedlichen (z. B. ansteigenden) Zeitverzögerungen durchgeführt wird, um dadurch das zeitliche Verhalten der Reaktion bzw. die zeitliche Veränderung der Struktur der biologischen Proben 1 zu untersuchen. Zur Steuerung der Zeitverzögerung kann die Vitrifizierungsvorrichtung 10 eine Steuereinrichtung 18 umfassen. Die Steuereinrichtung 18 kann zum Betätigen der Transfereinrichtung 12 und/oder des zumindest einen Flüssigkeitsdispensers 13 (z. B. mit einer einstellbaren Zeitverzögerung) eingerichtet sein. D. h. mittels der Steuereinrichtung 18 kann vorzugsweise ein Zeitpunkt des Zuführens der Flüssigkeit mittels des zumindest einen Flüssigkeitsdispensers 13 und/oder ein Zeitpunkt des Startens der Bewegung von der Ausgangsposition P₁ in die Freigabeposition P₂ steuerbar sein. Weiterhin kann die Steuereinrichtung 18 auch zum Betätigen der Auslöseeinrichtung 12b, der Bestrahlungseinrichtung 15, der Konditioniereinrichtung 16 und/oder der Makrokamera 19 eingerichtet sein. In einer bevorzugten Ausführungsform umfasst die Steuereinrichtung 18 ferner auch eine Sicherheitsvorrichtung 18a, die ausgebildet ist, ein Ausfahren der Transfereinrichtung 12 bei einem Wechsel des Probenhalters 2 zu blockieren.

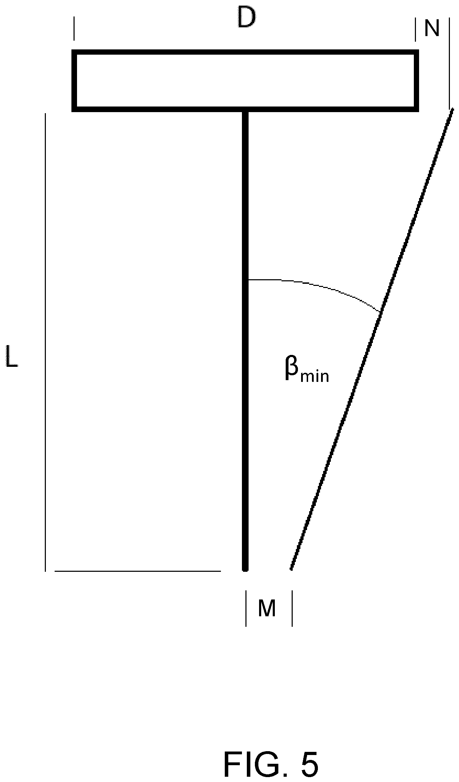

Figur 5 zeigt eine schematische Darstellung der geometrischen Zusammenhänge zwischen dem Probenhalter 2 und einem Flüssigkeitsdispenser 12 zur Festlegung eines Grenzwinkels βₘᵢₙ für einen Neigungswinkel β des Transferpads T. Wie vorstehend erwähnt, kann der Transferpfad T in einer Ausführungsform geneigt zur Trägerachse A des Probenhalters 2 verlaufen. Um dabei auf vorteilhafte Weise beim Transferieren des Probenträgers 2 mit der biologischen Probe 1 Kollisionen mit dem Flüssigkeitsdispenser 12 zu vermeiden, kann der Transferpfad T zur Trägerachse A um einen Neigungswinkel β geneigt verlaufen, der größer als ein Grenzwinkel βₘᵢₙ ist. D. h., der Neigungswinkel β soll hier bevorzugt zumindest einen Wert von βₘᵢₙ aufweisen. Dieser Grenzwinkel βₘᵢₙ kann hierbei durch den Zusammenhang βₘᵢₙ= arctan((D/2-M+N)/L) gegeben sein. D bezeichnet dabei eine laterale Ausdehnung des Sockels 2a. Beispielsweise kann D ein Durchmesser des Sockels 2a sein. Die laterale Ausdehnung soll bevorzugt eine Ausdehnung des Sockels 2a in einer Richtung senkrecht zur Trägerachse A bezeichnen. M bezeichnet einen vorgegebenen maximalen Abstand zwischen dem Flüssigkeitsdispenser 13 und der biologischen Probe 1. Beispielsweise kann M einen Wert von 1 mm aufweisen. Der vorgegebene maximale Abstand zwischen dem Flüssigkeitsdispenser 13 und der biologischen Probe 1 kann durch die Performance bzw. Zielgenauigkeit des Flüssigkeitsdispensers 13 gegeben sein. N bezeichnet einen vorgegebenen Sicherheitsabstand zwischen dem Flüssigkeitsdispenser 13 und dem Sockel 2a. Beispielsweise können hierüber mögliche Ungenauigkeiten bei der Positionierung berücksichtigt werden. L bezeichnet eine Länge des Stifts 2b. Als Länge des Stifts soll hierbei bevorzugt die Ausdehnung zwischen dem freien (zweiten) Stiftende und dem Sockel 2a sein.

Figur 6 zeigt eine schematische Darstellung einer Vitrifizierungsvorrichtung 10 gemäß einer weiteren Ausführungsform. Figur 6 dient dabei in erster Linie zur Veranschaulichung des alternativen Transfermechanismus, weshalb auf die Darstellung eine Vielzahl der möglichen weiteren optionalen Komponenten, z. B. die Bestrahlungseinrichtung 15, die Konditioniereinrichtung 16 etc., verzichtet wurde. Im Gegensatz zu der vorstehend im Zusammenhang mit den Figuren 2 bis 5 beschrieben Ausführungsform ist hier vorgesehen, dass der Transferpfad T entlang eines Kreisbogens verläuft (vgl. gestrichelte Linie in Figur 6). Wie beispielhaft dargestellt ist, kann der Transferpfad T dabei im Wesentlichen entlang eines Viertelkreisbogens verlaufen. D. h., die Trägerachse T kann in der Freigabeposition P₂ um 90° gedreht zur Trägerachse T in der Ausgangsposition P₁ orientiert sein. Bevorzugt ist die Trägerachse A in der Ausgangsposition P₁ dabei senkrecht zur Schwerkraftrichtung S orientiert. Die Trägerachse A kann jedoch auch schräg zur Schwerkraftrichtung S orientiert sein. Weiterhin kann das Bewegen des Probenträgers 2 mit der biologischen Probe 1 von der Ausgangsposition P₁ in die Freigabeposition P₂ ausschließlich eine Rotationsbewegung umfassen. Hierzu kann die Transfereinrichtung 12 bspw. über entsprechende Aktoren verfügen, um eine entsprechende Bewegung des Probenträgers 2 mit der biologischen Probe 1 zu ermöglichen. Beispielsweise kann in der vorliegenden Ausführungsform die Transfereinrichtung 12 einen Servomotor umfassen (nicht dargestellt), wobei die Halterung 12a, z. B. über eine entsprechende Verlängerung, an den Rotor des Servomotors bewegungsgekoppelt sein kann.

Obwohl die Erfindung unter Bezugnahme auf bestimmte Ausführungsbeispiele beschrieben worden ist, ist es für einen Fachmann ersichtlich, dass verschiedene Änderungen ausgeführt werden können und Äquivalente als Ersatz verwendet werden können, ohne den Bereich der Erfindung zu verlassen. Folglich soll die Erfindung nicht auf die offenbarten Ausführungsbei-spiele begrenzt sein, sondern soll alle Ausführungsbeispiele umfassen, die in den Bereich der beigefügten Patentansprüche fallen. Insbesondere beansprucht die Erfindung auch Schutz für den Gegenstand und die Merkmale der Unteransprüche unabhängig von den in Bezug genommenen Ansprüchen.

## Patentansprüche

1. Verfahren zum Vitrifizieren einer biologischen Probe (1), aufweisend die Schritte:
- Positionieren eines Probenträgers (2) mit der biologischen Probe (1) mittels einer Transfereinrichtung (12) in einer Ausgangsposition (P₁),
wobei der Probenträger (2) einen Sockel (2a) und einen, vom Sockel (2a) entlang einer Trägerachse (A) abstehenden, Stift (2b) aufweist und wobei die biologische Probe (1) am Stift (2b) beabstandet vom Sockel (2a) befestigt ist;
- Zuführen einer Flüssigkeit zu der biologischen Probe (1) in der Ausgangsposition (P₁) mittels eines Flüssigkeitsdispensers (13);
- Bewegen des Probenträgers (2) mit der biologischen Probe (1) mittels der Transfereinrichtung (12) entlang eines vorbestimmten Transferpfads (T) von der Ausgangsposition (P₁) in eine Freigabeposition (P₂), wobei die biologische Probe (1) in der Freigabeposition (P₂) in oder angrenzend zu einem verflüssigten Gas (3) angeordnet ist;
**dadurch gekennzeichnet, dass** der Transferpfad (T) zur Trägerachse (A) geneigt verläuft.

2. Verfahren nach einem Anspruch 1, wobei:
a) der Transferpfad (T) gerade ist und/oder ohne Richtungswechsel verläuft; und/oder
b) das Bewegen des Probenträgers (2) mit der biologischen Probe (1) von der Ausgangsposition (P₁) in die Freigabeposition (P₂) ausschließlich eine Translationsbewegung umfasst.

3. Verfahren nach einem der vorherigen Ansprüche, wobei der Transferpfad (T) zur Trägerachse (A) um einen Neigungswinkel (β) geneigt verläuft, wobei der Neigungswinkel (β):
a) ein Winkel zwischen 5° und 45°, vorzugsweise zwischen 10° und 30°, besonders bevorzugt zwischen 10° und 20° ist; und/oder
b) größer als ein Grenzwinkel, βₘᵢₙ, ist, wobei der Grenzwinkel durch βₘᵢₙ = arctan((D/2-M+N)/L) bestimmt ist, wobei D eine laterale Ausdehnung des Sockels (2a), M ein vorgegebener maximaler Abstand zwischen dem Flüssigkeitsdispenser (13) und der biologischen Probe (1), N ein vorgegebener Sicherheitsabstand zwischen dem Flüssigkeitsdispenser (13) und dem Sockel (2a) und L eine Länge des Stifts (2b) sind.

4. Verfahren nach einem der vorherigen Ansprüche, wobei die Trägerachse (A) in der Ausgangsposition (P₁) entlang der Schwerkraftrichtung (S) orientiert ist.

5. Verfahren nach einem der vorherigen Ansprüche, wobei der Transferpfad (T) derart verläuft, dass sich der Probenträger (2) und/oder die Transfereinrichtung (12) beim Bewegen von der Ausgangsposition (P₁) in die Freigabeposition (P₂) kollisionsfrei am Flüssigkeitsdispenser (13) vorbei bewegen.

6. Verfahren nach einem der vorherigen Ansprüche, wobei zwischen dem Zuführen der Flüssigkeit in der Ausgangsposition (P₁) und dem Bewegen des Probenträgers (2) mit der biologischen Probe (1) von der Ausgangsposition (P₁) in die Freigabeposition (P₂) eine vorbestimmte Zeitspanne gewartet wird und/oder eine Lage des Flüssigkeitsdispensers (13) nicht verändert wird.

7. Verfahren nach einem der vorherigen Ansprüche, ferner aufweisend:
- optisches Anregen der biologischen Probe (1) in der Ausgangsposition (P₁) mit zumindest einem Lichtimpuls mittels einer Bestrahlungseinrichtung (15).

8. Verfahren nach einem der vorherigen Ansprüche, ferner aufweisend:
- Bereitstellen einer vorbestimmten Feuchte und/oder Temperatur in einer Umgebung der Probe (1) in der Ausgangsposition (P₁) mittels einer Konditioniereinrichtung (16).

9. Verfahren nach einem der vorherigen Ansprüche, ferner aufweisend:
- Freigeben des Probenträgers (2) mit der biologischen Probe (1) aus der Transfereinrichtung (12) in der Freigabeposition (P₂) mittels einer Auslöseeinrichtung (12b) der Transfereinrichtung (12), sodass der Probenträger (2) schwerkraftvermittelt aus der Transfereinrichtung (12) fallen kann.

10. Verfahren zum Vitrifizieren einer biologischen Probe (1), aufweisend die Schritte:
- Positionieren eines Probenträgers (2) mit der biologischen Probe (1) mittels einer Transfereinrichtung (12) in einer Ausgangsposition (P₁),
wobei der Probenträger (2) einen Sockel (2a) und einen, vom Sockel (2a) entlang einer Trägerachse (A) abstehenden, Stift (2b) aufweist und wobei die biologische Probe (1) am Stift (2b) beabstandet vom Sockel (2a) befestigt ist;
- Zuführen einer Flüssigkeit zu der biologischen Probe (1) in der Ausgangsposition (P₁) mittels eines Flüssigkeitsdispensers (13);
- Bewegen des Probenträgers (2) mit der biologischen Probe (1) mittels der Transfereinrichtung (12) entlang eines vorbestimmten Transferpfads (T) von der Ausgangsposition (P₁) in eine Freigabeposition (P₂),
wobei die biologische Probe (1) in der Freigabeposition (P₂) in oder angrenzend zu einem verflüssigen Gas (3) angeordnet ist;
**dadurch gekennzeichnet, dass** der Transferpfad (T) entlang eines Kreisbogens verläuft.

11. Verfahren nach Anspruch 10, wobei:
a) der Transferpfad (T) im Wesentlichen entlang eines Viertelkreisbogens verläuft; und/oder
b) das Bewegen des Probenträgers (2) mit der biologischen Probe (1) von der Ausgangsposition (P₁) in die Freigabeposition (P₂) ausschließlich eine Rotationsbewegung umfasst.

12. Verfahren nach Anspruch 10 oder 11, wobei die Trägerachse (A) in der Ausgangsposition (P₁) im Wesentlichen senkrecht zur Schwerkraftrichtung (S) orientiert ist.

13. Verfahren nach einem der Ansprüche 10 bis 12, wobei das Verfahren ferner die Merkmale nach einem der Ansprüche 5 bis 9 umfasst.

14. Vitrifizierungsvorrichtung (10), die zur Durchführung eines Verfahren nach einem der vorherigen Ansprüche ausgebildet ist, aufweisend:
einen Behälter (11) zur Aufnahme des verflüssigten Gases (3);
eine Transfereinrichtung (12) mit einer Halterung (12a) zur Aufnahme des Probenträgers (2) mit der biologischen Probe (1),
wobei die Transfereinrichtung (12) ausgebildet ist, den in der Halterung (12a) aufgenommenen Probenträger (2) mit der biologischen Probe (1) in der Ausgangsposition (P₁) zu positionieren und entlang des vorbestimmten Transferpfads (T) von der Ausgangsposition (P₁) in die Freigabeposition (P₂) zu bewegen; und
einen Flüssigkeitsdispenser (13) zum Zuführen der Flüssigkeit zu der biologischen Probe (1) in der Ausgangsposition (P₁).

15. Vitrifizierungsvorrichtung (10) nach Anspruch 14, ferner aufweisend:
eine Bestrahlungseinrichtung (15), die angeordnet ist, die biologische Probe (1) in der Ausgangsposition (P₁) mittels zumindest eines Lichtimpulses optisch anzuregen; und/oder
eine Konditioniereinrichtung (16), die zur Bereitstellung einer vorbestimmten Feuchte und/oder Temperatur in einer Umgebung der biologischen Probe (1) in der Ausgangsposition (P₁) eingerichtet ist; und/oder
eine Auslöseeinrichtung (12b), mittels derer der Probenträger (2) mit der biologischen Probe (1) für eine Entnahme des Probenträger (2) aus der Transfereinrichtung (12) und/oder der Halterung (12a) freigebbar ist; und/oder
eine Lagereinrichtung (17), die in dem Behälter (12) angeordnet ist und die zumindest eine Aufnahme (17a) aufweist, in die der Probenträger (2) mit der biologischen Probe (1) aufnehmbar ist, wobei die Lagereinrichtung (17) vorzugsweise derart angeordnet ist, dass, wenn sich der Probenträger (2) mit der biologischen Probe (1) in der Freigabeposition (P₂) befindet, die zumindest eine Aufnahme (17a) unterhalb des Probenträgers (2) mit der biologischen Probe (1) angeordnet ist.
